(19) Europäisches Patentamt
European Patent Office
Office européen des brevets

(11) EP 1 389 613 A1

(12) **EUROPEAN PATENT APPLICATION**
published in accordance with Art. 158(3) EPC

(43) Date of publication:
**18.02.2004 Bulletin 2004/08**

(21) Application number: **02720607.7**

(22) Date of filing: **25.04.2002**

(51) Int Cl.[7]: **C07C 323/42**, C07C 317/28, C07C 317/40, C07D 213/64, A01N 37/22, A01N 41/10, A01N 43/40

(86) International application number:
**PCT/JP2002/004172**

(87) International publication number:
**WO 2002/088075 (07.11.2002 Gazette 2002/45)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **26.04.2001 JP 2001130404**

(71) Applicant: **Nihon Nohyaku Co., Ltd.**
**Tokyo 103-8236 (JP)**

(72) Inventors:
- **HARAYAMA, Hiroto**
  **Kawachinagano-shi, Osaka 586-0022 (JP)**
- **TOHNISHI, Masanori**
  **Sakai-shi, Osaka 599-8241 (JP)**
- **MORIMOTO, Masayuki**
  **Kawachinagano-shi, Osaka 586-0024 (JP)**
- **FUJIOKA, Shinsuke**
  **Kawachinagano-shi, Osaka 586-0037 (JP)**
- **FISCHER, Rüdiger**
  **50259 Pulheim (DE)**
- **KONZE, Jörg**
  **51147 Cologne (DE)**

(74) Representative: **Grünecker, Kinkeldey, Stockmair & Schwanhäusser Anwaltssozietät**
**Maximilianstrasse 58**
**80538 München (DE)**

(54) **PHTHALAMIDE DERIVATIVES, INSECTICIDES FOR AGRICULTURAL AND HORTICULTURAL USE AND METHOD FOR APPLICATION THEREOF**

(57) The present invention relates to a phthalamide derivative represented by general formula (I) :

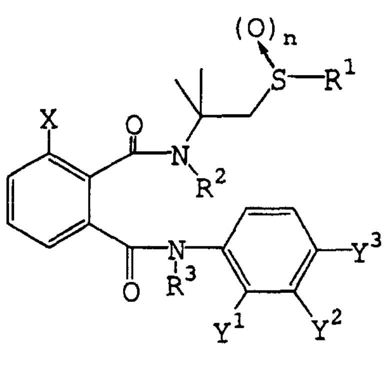

(I)

wherein each of $R^1$, $R^2$ and $R^3$, which may be the same or different, is a hydrogen atom, a ($C_1$-$C_6$)alkyl group, a ($C_3$-$C_6$)alkenyl group or a ($C_3$-$C_6$)alkynyl group; each of X and $Y^3$, which may be the same or different, is a halogen atom; each of $Y^1$ and $Y^2$, which may be the same or different, is a hydrogen atom, a halogen atom, a nitro group, a cyano group, a ($C_1$-$C_6$)alkyl group or the like; and n is an integer of 0 to 2; and an agricultural and horticultural insecticide containing said compound as an active ingredient and a method of using the same.

EP 1 389 613 A1

## Description

### TECHNICAL FIELD

The present invention relates to phthalamide derivatives, agricultural and horticultural insecticides containing any of said compounds as an active ingredient, and their usage.

### BACKGROUND ART

JP-A-11-240857 and JP-A-2001-131141 describe compounds analogous to the phthalamide derivatives of the present invention as useful as agricultural and horticultural insecticides. These references, however, do not describe working examples, physical properties and the like with respect to the compounds of the present invention.

The production of agricultural and horticultural crops and the like is still badly damaged by insect pests and the like, and the development of a novel agricultural and horticultural chemical, in particular, agricultural and horticultural insecticide is desired because of, for example, the appearance of insect pests resistant to existing chemicals. In addition, because of the increased population of aged farmers, and the like, various labor-saving application methods are desired and the development of an agricultural and horticultural chemical having properties suitable for the application methods is desired.

### DISCLOSURE OF THE INVENTION

The present inventors earnestly investigated in order to develop a novel agricultural and horticultural chemical, and consequently found that the phthalamide derivatives represented by general formula (I) of the present invention are novel compounds not known in any literature and are excellent agricultural and horticultural insecticides which exhibit an excellent insecticidal effect at a lower dosage as compared with the compounds disclosed in JP-A-11-240857 and JP-A-2001-131141 and can be absorbed and transferred through roots to a high degree particularly when applied to soil or the like, whereby the present invention has been accomplished.

That is, the present invention relates to phthalamide derivatives represented by general formula (I) :

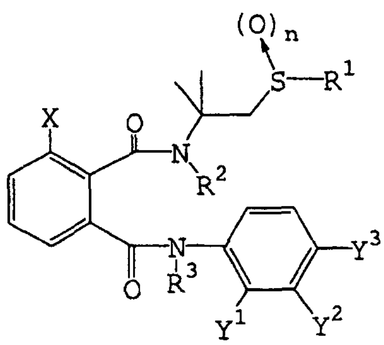

(I)

wherein each of $R^1$, $R^2$ and $R^3$, which may be the same or different, is a hydrogen atom, a $(C_1-C_6)$alkyl group, a $(C_3-C_6)$ alkenyl group or a $(C_3-C_6)$alkynyl group; each of X and $Y^3$, which may be the same or different, is a halogen atom; each of $Y^1$ and $Y^2$, which may be the same or different, is a hydrogen atom, a halogen atom, a nitro group, a cyano group, a $(C_1-C_6)$alkyl group, halo$(C_1-C_6)$alkyl group, a $(C_2-C_6)$alkenyl group, a halo$(C_2-C_6)$alkenyl group, a $(C_2-C_6)$ alkynyl group, a halo$(C_2-C_6)$alkynyl group, a $(C_3-C_6)$cycloalkyl group, a halo$(C_3-C_6)$cycloalkyl group, a $(C_1-C_6)$alkoxy group, halo$(C_1-C_6)$alkoxy group, a $(C_1-C_6)$alkylthio group, halo$(C_1-C_6)$alkylthio group, a $(C_1-C_6)$alkylsulfinyl group, halo $(C_1-C_6)$alkylsulfinyl group, a $(C_1-C_6)$alkylsulfonyl group, halo$(C_1-C_6)$alkylsulfonyl group, a mono$(C_1-C_6)$alkylamino group or a di$(C_1-C_6)$alkylamino group whose $(C_1-C_6)$alkyl groups may be the same or different; and n is an integer of 0 to 2; an agricultural and horticultural insecticide containing said compound as an active ingredient, and a method of using the same.

The compounds of the present invention exhibit an excellent insecticidal effect at a low dosage and can be absorbed and transferred through roots to a high degree particularly when applied to soil or the like.

MODE FOR CARRYING OUT THE INVENTION

In the definition of general formula (I) for the phthalamide derivative of the present invention, the term "halogen atom" means a chlorine atom, a bromine atom, an iodine atom or a fluorine atom. The term "($C_1$-$C_6$)alkyl" means a linear or branched alkyl group of 1 to 6 carbon atoms, such as methyl, ethyl, n-propyl, i-propyl, n-butyl, i-butyl, s-butyl, t-butyl, n-pentyl, n-hexyl or the like. The term "halo($C_1$-$C_6$)alkyl" means a substituted linear or branched alkyl group of 1 to 6 carbon atoms having as the substituent(s) one or more halogen atoms which may be the same or different. The term "($C_3$-$C_6$)cycloalkyl" means, for example, a cyclic alkyl group of 3 to 6 carbon atoms, such as cyclopropyl, cyclobutyl, cyclopentyl, cyclohexyl or the like.

The phthalamide derivative of general formula (I) of the present invention contains one or more asymmetric centers in its structural formula in some cases and has two or more optical isomers and diastereomers in some cases. The present invention also includes all of the individual optical isomers and mixtures consisting of these isomers in any ratio. The phthalamide derivative of general formula (I) of the present invention has two geometrical isomers due to a carbon-carbon double bond in its structural formula in some cases. The present invention also includes the individual geometrical isomers and mixtures consisting of these isomers in any ratio.

Preferable compounds as the phthalamide derivative of general formula (I) of the present invention are, for example, phthalamide derivatives in which $R^1$ is a ($C_1$-$C_6$) alkyl group, each of $R^2$ and $R^3$ is a hydrogen atom, each of X and $Y^3$, which may be the same or different, is a halogen atom, $Y^1$ is a halogen atom or a ($C_1$-$C_6$)alkyl group, $Y^2$ is a halogen atom, and n is an integer of 0 to 2. More preferable compounds are phthalamide derivatives in which $R^1$ is a methyl group, each of $R^2$ and $R^3$ is a hydrogen atom, X is an iodine atom, each of $Y^1$, $Y^2$ and $Y^3$, which may be the same or different, is a halogen atom, and n is an integer of 0 to 2.

A typical production process according to the present invention is schematically shown below but it is not intended in any way to limit the scope of the present invention.

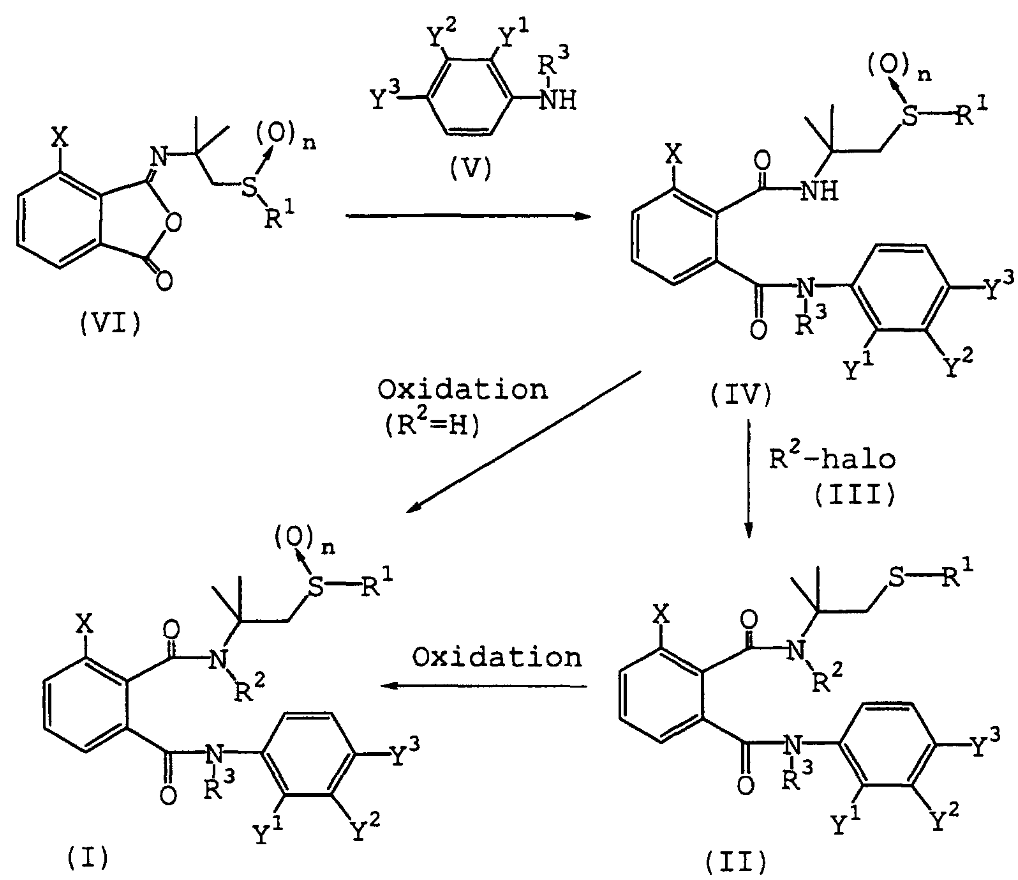


wherein $R^1$, $R^2$, $R^3$, X, $Y^1$, $Y^2$, $Y^3$ and n are as defined above, and halo is a halogen atom.

The phthalamide derivative of general formula (I) can be produced by allowing a phthalisoimide derivative of general formula (VI) to react with an aniline derivative of general formula (V) in an inert solvent in the presence or

absence of an acid or a base to obtain a phthalamide derivative of general formula (IV), allowing the phthalamide derivative (IV) to react with a halide of general formula (III) in the presence of a dehydrohalogenating agent and an inert solvent to obtain a phthalamide derivative of general formula (II), and then allowing the phthalamide derivative (II) to react with an oxidizing agent in the presence of an inert solvent.

When $R^2$ is a hydrogen atom, the phthalamide derivative of general formula (I) can be produced by allowing a phthalamide derivative of general formula (IV) to react with an oxidizing agent in the presence of an inert solvent, without a step of obtaining a phthalamide derivative of general formula (II).

The phthalisoimide derivative of general formula (VI), i.e., the starting compound used in the present invention can be produced according to either of the production processes disclosed in JP-A-11-240857 and JP-A-2001-131141.

(1). General formula (VI) → general formula (IV)

In the case of this reaction, the desired compound can be produced according to the process described in J. Med. Chem., 10, 982 (1967).

The inert solvent used in this reaction includes, for example, tetrahydrofuran, diethyl ether, methyl t-butyl ether, dioxane, chloroform, methylene chloride, chlorobenzene, toluene, acetonitrile, ethyl acetate and butyl acetate.

The acid usable in the reaction includes, for example, organic acids such as acetic acid, trifluoroacetic acid, etc.; and inorganic acids such as hydrochloric acid, sulfuric acid, etc. As to the amount of the acid used, the acid may be used in an amount properly chosen in the range of a catalytic amount to excess moles per mole of the phthalisoimide derivative of general formula (VI). The base includes, for example, organic bases such as triethylamine, pyridine, etc.; and inorganic bases such as potassium carbonate, sodium hydrogencarbonate, sodium carbonate, sodium hydroxide, etc. As to the amount of the base used, the base may be used in an amount properly chosen in the range of a catalytic amount to excess moles per mole of the phthalisoimide derivative of general formula (VI).

As to the reaction temperature, the reaction can be carried out at 0°C to the boiling point of the inert solvent used. Although the reaction time is varied depending on the scale of reaction, the reaction temperature, etc., it ranges from several minutes to 48 hours. After completion of the reaction, the desired compound is isolated from the reaction system containing the desired compound by a conventional method, and if necessary, purified by recrystallization, column chromatography, etc., whereby the desired compound can be produced.

(2). General formula (IV) → general formula (II)

As the inert solvent used in this reaction, any inert solvent may be used so long as it does not markedly inhibit the progress of the reaction. There can be exemplified inert solvents including aromatic hydrocarbons such as benzene, toluene, xylene, etc.; halogenated aromatic hydrocarbons such as fluorobenzene, chlorobenzene, dichlorobenzene, etc.; halogenated hydrocarbons such as methylene chloride, chloroform, carbon tetrachloride, etc.; acyclic or cyclic ethers such as diethyl ether, dioxane, tetrahydrofuran, etc.; esters such as ethyl acetate, etc.; amides such as dimethylformamide, dimethylacetamide, etc.; acids such as acetic acid, etc.; dimethyl sulfoxide; and 1,3-dimethyl-2-imidazolidinone. These inert solvents may be used singly or as a mixture of two or more thereof.

The dehydrohalogenating agent includes, for example, organic bases such as triethylamine, pyridine, etc.; and inorganic bases such as potassium carbonate, sodium hydrogencarbonate, sodium carbonate, sodium hydroxide, etc. Since the reaction is an equimolar reaction, it is sufficient that the reactants are used in equimolar amounts, though either of them may be used in excess.

As to the reaction temperature, the reaction may be carried out at room temperature to the reflux temperature of the inert solvent used. Although the reaction time is varied depending on the scale of reaction, the reaction temperature, etc., it may be properly chosen in the range of several minutes to 48 hours.

After completion of the reaction, the desired compound is isolated from the reaction system containing the desired compound by a conventional method, and if necessary, purified by recrystallization, column chromatography, etc., whereby the desired compound can be produced. It is also possible to subject the desired compound to the subsequent reaction step without isolation from the reaction system.

(3). General formula (II) or (IV) → general formula (I)

The inert solvent used in this reaction includes, for example, halogenated hydrocarbons such as methylene chloride, chloroform, etc.; aromatic hydrocarbons such as toluene, xylene, etc.; halogenated aromatic hydrocarbons such as fluorobenzene, chlorobenzene, dichlorobenzene, etc.; acids such as acetic acid, etc.; and alcohols such as methanol, ethanol, propanol, etc.

The oxidizing agent includes, for example, m-chloroperbenzoic acid, peracetic acid, potassium metaperiodate, potassium hydrogenpersulfate (oxone), and hydrogen peroxide. As to the amount of the oxidizing agent used,

the oxidizing agent may be used in an amount properly chosen in the range of 0.5 to 3 equivalents per equivalent of the phthalamide derivative of general formula (II) or (IV).

As to the reaction temperature, the reaction may be carried out in the range of -50°C to the boiling point of the inert solvent used. Although the reaction time is varied depending on the reaction temperature, the scale of reaction, etc., it ranges from several minutes to 24 hours.

After completion of the reaction, the desired compound is isolated from the reaction system containing the desired compound by a conventional method, and if necessary, purified by recrystallization, column chromatography, etc., whereby the desired compound can be produced.

Typical compounds as the phthalamide derivative of general formula (I) of the present invention are listed below in Table 1 and Table 2 but they are not intended in any way to limit the scope of the present invention.

General formula (I)

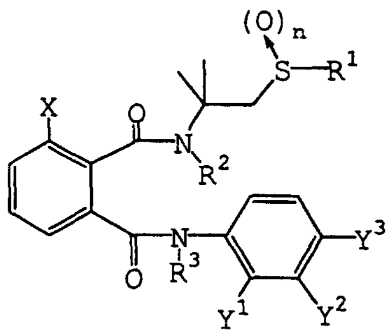

(I)

Table 1

| ($R^1$ = $CH_3$, $R^2$ = H) | | | | | | | |
|---|---|---|---|---|---|---|---|
| No. | $R^3$ | X | $Y^1$ | $Y^2$ | $Y^3$ | n | Melting point (°C) |
| 1 | H | F | Cl | Cl | Cl | 0 | 199-201 |
| 2 | H | Cl | Cl | Cl | Cl | 0 | 189-190 |
| 3 | H | Cl | Cl | Cl | Cl | 1 | |
| 4 | H | Cl | Cl | Cl | Cl | 2 | 198-201 |
| 5 | H | Br | Cl | Cl | Cl | 0 | 178-181 |
| 6 | H | Br | Cl | Cl | Cl | 1 | |
| 7 | H | Br | Cl | Cl | Cl | 2 | 227-228 |
| 8 | H | I | H | Cl | F | 0 | |
| 9 | H | I | H | Cl | Cl | 0 | |
| 10 | H | I | H | Cl | Br | 0 | |
| 11 | H | I | H | Cl | I | 0 | |
| 12 | H | I | H | Br | Cl | 0 | 209-211 |
| 13 | H | I | H | Br | Br | 0 | |
| 14 | H | I | H | Br | I | 0 | |
| 15 | H | I | H | I | Cl | 0 | 204-206 |
| 16 | H | I | H | I | Br | 0 | |
| 17 | H | I | H | I | I | 0 | |
| 18 | H | I | H | $CF_3$ | F | 0 | |
| 19 | H | I | H | $CF_3$ | Cl | 0 | 180-182 |
| 20 | H | I | H | $CF_3$ | Cl | 1 | 129-134 |
| 21 | H | I | H | $CF_3$ | Cl | 2 | |

Table 1 (continued)

| ($R^1 = CH_3$, $R^2 = H$) | | | | | | | |
|---|---|---|---|---|---|---|---|
| No. | $R^3$ | X | $Y^1$ | $Y^2$ | $Y^3$ | n | Melting point (°C) |
| 22 | H | I | H | $CF_3$ | Br | 0 | |
| 23 | H | I | H | $CF_3$ | I | 0 | 88-90 |
| 24 | H | I | $CH_3$ | H | Cl | 0 | 178-180 |
| 25 | H | I | $CH_3$ | H | Cl | 1 | 112-113 |
| 26 | H | I | $CH_3$ | H | Cl | 2 | 106-107 |
| 27 | H | I | $CH_3$ | H | Br | 0 | 172-173 |
| 28 | H | I | $CH_3$ | H | Br | 1 | 96-98 |
| 29 | H | I | $CH_3$ | H | Br | 2 | 118-120 |
| 30 | H | I | $CH_3$ | H | I | 0 | 155-157 |
| 31 | H | I | $CH_3$ | H | I | 1 | 99-102 |
| 32 | H | I | $CH_3$ | H | I | 2 | 116-118 |
| 33 | H | I | $CH_3$ | F | F | 0 | |
| 34 | H | I | $CH_3$ | F | Cl | 0 | |
| 35 | H | I | $CH_3$ | F | Br | 0 | |
| 36 | H | I | $CH_3$ | F | I | 0 | |
| 37 | H | I | $CH_3$ | Cl | F | 0 | |
| 38 | H | I | $CH_3$ | Cl | Cl | 0 | |
| 39 | H | I | $CH_3$ | Cl | Cl | 1 | |
| 40 | H | I | $CH_3$ | Cl | Cl | 2 | |
| 41 | H | I | $CH_3$ | Cl | Br | 0 | |
| 42 | H | I | $CH_3$ | Cl | I | 0 | 202-204 |
| 43 | H | I | $CH_3$ | Cl | I | 1 | 102-107 |
| 44 | H | I | $CH_3$ | Cl | I | 2 | 132-135 |
| 45 | H | I | $CH_3$ | Br | Cl | 0 | |
| 46 | H | I | $CH_3$ | Br | Br | 0 | |
| 47 | H | I | $CH_3$ | Br | I | 0 | |
| 48 | H | I | $CH_3$ | I | Cl | 0 | |
| 49 | H | I | $CH_3$ | I | Br | 0 | |
| 50 | H | I | $CH_3$ | I | I | 0 | |
| 51 | H | I | $CH_3$ | $CF_3$ | Cl | 0 | |
| 52 | H | I | $CH_3$ | $CF_3$ | Br | 0 | |
| 53 | H | I | $CH_3$ | $CF_3$ | I | 0 | |
| 54 | H | I | $CF_3$ | H | Cl | 0 | |
| 55 | H | I | $CF_3$ | H | Br | 0 | |
| 56 | H | I | $CF_3$ | H | I | 0 | 154-155 |
| 57 | H | I | $CF_3$ | H | I | 1 | 120-125 |
| 58 | H | I | $CF_3$ | H | I | 2 | 115-117 |
| 59 | H | I | $CF_3$ | Cl | Cl | 0 | |
| 60 | H | I | $CF_3$ | Cl | Cl | 1 | |
| 61 | H | I | $CF_3$ | Cl | Cl | 2 | |
| 62 | H | I | $CF_3$ | Cl | Br | 0 | |
| 63 | H | I | $CF_3$ | Cl | I | 0 | |
| 64 | H | I | CN | H | Cl | 0 | 144-148 |
| 65 | H | I | CN | H | Cl | 1 | 118-121 |
| 66 | H | I | CN | H | Cl | 2 | 114-117 |
| 67 | H | I | CN | H | Br | 0 | |
| 68 | H | I | CN | H | I | 0 | |

Table 1 (continued)

| ($R^1 = CH_3$, $R^2 = H$) | | | | | | | |
|---|---|---|---|---|---|---|---|
| No. | $R^3$ | X | $Y^1$ | $Y^2$ | $Y^3$ | n | Melting point (°C) |
| 69 | H | I | CN | Cl | Cl | 0 | |
| 70 | H | I | CN | Cl | Cl | 1 | |
| 71 | H | I | CN | Cl | Cl | 2 | |
| 72 | H | I | CN | Cl | Br | 0 | |
| 73 | H | I | CN | Cl | I | 0 | |
| 74 | H | I | F | H | F | 0 | 146-148 |
| 75 | H | I | F | H | F | 1 | 85-88 |
| 76 | H | I | F | H | F | 2 | |
| 77 | H | I | F | H | Cl | 0 | |
| 78 | H | I | F | H | Br | 0 | |
| 79 | H | I | F | H | I | 0 | |
| 80 | H | I | F | F | F | 0 | 157-159 |
| 81 | H | I | F | F | Cl | 0 | |
| 82 | H | I | F | F | Br | 0 | |
| 83 | H | I | F | F | I | 0 | |
| 84 | H | I | F | Cl | F | 0 | 169-171 |
| 85 | H | I | F | Cl | Cl | 0 | |
| 86 | H | I | F | Cl | Cl | 1 | |
| 87 | H | I | F | Cl | Cl | 2 | |
| 88 | H | I | F | Cl | Br | 0 | |
| 89 | H | I | F | Cl | I | 0 | |
| 90 | H | I | F | Br | F | 0 | |
| 91 | H | I | F | Br | Cl | 0 | |
| 92 | H | I | F | Br | Br | 0 | |
| 93 | H | I | F | Br | I | 0 | |
| 94 | H | I | F | I | F | 0 | |
| 95 | H | I | F | I | Cl | 0 | |
| 96 | H | I | F | I | Br | 0 | |
| 97 | H | I | F | I | I | 0 | |
| 98 | H | I | Cl | H | F | 0 | |
| 99 | H | I | Cl | H | Cl | 0 | 155-157 |
| 100 | H | I | Cl | H | Cl | 1 | 82-86 |
| 101 | H | I | Cl | H | Cl | 2 | 109-111 |
| 102 | H | I | Cl | H | Br | 0 | 149-151 |
| 103 | H | I | Cl | H | Br | 1 | 85-89 |
| 104 | H | I | Cl | H | Br | 2 | 118-120 |
| 105 | H | I | Cl | H | I | 0 | 133-135 |
| 106 | H | I | Cl | H | I | 1 | 84-88 |
| 107 | H | I | Cl | H | I | 2 | 108-109 |
| 108 | H | I | Cl | F | F | 0 | |
| 109 | H | I | Cl | F | Cl | 0 | |
| 110 | H | I | Cl | F | Cl | 1 | |
| 111 | H | I | Cl | F | Cl | 2 | |
| 112 | H | I | Cl | F | Br | 0 | |
| 113 | H | I | Cl | F | I | 0 | |
| 114 | H | I | Cl | Cl | F | 0 | |
| 115 | H | I | Cl | Cl | F | 1 | |

Table 1 (continued)

| ($R^1 = CH_3$, $R^2$ = H) | | | | | | | |
|---|---|---|---|---|---|---|---|
| No. | $R^3$ | X | $Y^1$ | $Y^2$ | $Y^3$ | n | Melting point (°C) |
| 116 | H | I | Cl | Cl | F | 2 | |
| 117 | H | I | Cl | Cl | Cl | 0 | 175-176 |
| 118 | H | I | Cl | Cl | Cl | 1 | 94-98 |
| 119 | H | I | Cl | Cl | Cl | 2 | 239-241 |
| 120 | H | I | Cl | Cl | Br | 0 | 183 |
| 121 | H | I | Cl | Cl | Br | 1 | 95-100 |
| 122 | H | I | Cl | Cl | Br | 2 | 243 |
| 123 | H | I | Cl | Cl | I | 0 | 183-185 |
| 124 | H | I | Cl | Cl | I | 1 | 89-94 |
| 125 | H | I | Cl | Cl | I | 2 | 111-113 |
| 126 | H | I | Cl | Br | F | 0 | |
| 127 | H | I | Cl | Br | Cl | 0 | |
| 128 | H | I | Cl | Br | Cl | 1 | |
| 129 | H | I | Cl | Br | Cl | 2 | |
| 130 | H | I | Cl | Br | Br | 0 | |
| 131 | H | I | Cl | Br | I | 0 | |
| 132 | H | I | Cl | I | Cl | 0 | |
| 133 | H | I | Cl | I | Cl | 1 | |
| 134 | H | I | Cl | I | Cl | 2 | |
| 135 | H | I | Cl | I | Br | 0 | |
| 136 | H | I | Cl | I | I | 0 | |
| 137 | H | I | Cl | $CH_3$ | F | 0 | |
| 138 | H | I | Cl | $CH_3$ | Cl | 0 | 169-173 |
| 139 | H | I | Cl | $CH_3$ | Br | 0 | |
| 140 | H | I | Cl | $CH_3$ | I | 0 | |
| 141 | H | I | Cl | $CF_3$ | F | 0 | |
| 142 | H | I | Cl | $CF_3$ | Cl | 0 | 196-198 |
| 143 | H | I | Cl | $CF_3$ | Cl | 1 | 97-101 |
| 144 | H | I | Cl | $CF_3$ | Cl | 2 | 205-207 |
| 145 | H | I | Cl | $CF_3$ | Br | 0 | |
| 146 | H | I | Cl | $CF_3$ | I | 0 | |
| 147 | H | I | Cl | CN | Cl | 0 | 184-185 |
| 148 | H | I | Cl | CN | Cl | 1 | 92-97 |
| 149 | H | I | Cl | CN | Cl | 2 | 206-208 |
| 150 | H | I | Cl | CN | Br | 0 | |
| 151 | H | I | Cl | CN | I | 0 | |
| 152 | H | I | Cl | $OCHF_2$ | Cl | 0 | |
| 153 | H | I | Cl | $OCClF_2$ | Cl | 0 | 96-98 |
| 154 | H | I | Cl | $OCF_3$ | Cl | 0 | |
| 155 | H | I | Cl | $SCF_3$ | Cl | 0 | |
| 156 | H | I | Cl | $NO_2$ | Cl | 0 | |
| 157 | H | I | Br | H | F | 0 | |
| 158 | H | I | Br | H | Cl | 0 | |
| 159 | H | I | Br | H | Br | 0 | |
| 160 | H | I | Br | H | I | 0 | |
| 161 | H | I | Br | F | Cl | 0 | |
| 162 | H | I | Br | F | Br | 0 | |

Table 1 (continued)

| ($R^1 = CH_3$, $R^2 = H$) | | | | | | | |
|---|---|---|---|---|---|---|---|
| No. | $R^3$ | X | $Y^1$ | $Y^2$ | $Y^3$ | n | Melting point (°C) |
| 163 | H | I | Br | F | I | 0 | |
| 164 | H | I | Br | Cl | Cl | 0 | |
| 165 | H | I | Br | Cl | Cl | 1 | |
| 166 | H | I | Br | Cl | Cl | 2 | |
| 167 | H | I | Br | Cl | Br | 0 | |
| 168 | H | I | Br | Cl | I | 0 | |
| 169 | H | I | Br | Br | Cl | 0 | |
| 170 | H | I | Br | Br | Br | 0 | |
| 171 | H | I | Br | Br | I | 0 | |
| 172 | H | I | Br | I | Cl | 0 | |
| 173 | H | I | Br | I | Br | 0 | |
| 174 | H | I | Br | I | I | 0 | |
| 175 | H | I | I | H | F | 0 | 160-163 |
| 176 | H | I | I | H | Cl | 0 | |
| 177 | H | I | I | H | Br | 0 | |
| 178 | H | I | I | H | I | 0 | |
| 179 | H | I | I | F | Cl | 0 | |
| 180 | H | I | I | F | Br | 0 | |
| 181 | H | I | I | F | I | 0 | |
| 182 | H | I | I | Cl | Cl | 0 | |
| 183 | H | I | I | Cl | Cl | 1 | |
| 184 | H | I | I | Cl | Cl | 2 | |
| 185 | H | I | I | Cl | Br | 0 | |
| 186 | H | I | I | Cl | I | 0 | |
| 187 | H | I | I | Br | Cl | 0 | |
| 188 | H | I | I | Br | Br | 0 | |
| 189 | H | I | I | Br | I | 0 | |
| 190 | H | I | I | I | Cl | 0 | |
| 191 | H | I | I | I | Br | 0 | |
| 192 | H | I | I | I | I | 0 | |
| 193 | H | I | $OCH_3$ | H | Cl | 0 | |
| 194 | H | I | $OCH_3$ | H | Br | 0 | |
| 195 | H | I | $OCH_3$ | H | I | 0 | |
| 196 | H | I | $OCH_3$ | Cl | Cl | 0 | 107-109 |
| 197 | H | I | $OCH_3$ | Cl | Br | 0 | |
| 198 | H | I | $OCH_3$ | Cl | I | 0 | |
| 199 | H | I | $OCF_3$ | H | Cl | 0 | |
| 200 | H | I | $OCF_3$ | H | Br | 0 | |
| 201 | H | I | $OCF_3$ | H | I | 0 | 145-147 |
| 202 | H | I | $OCF_3$ | H | I | 1 | 98-102 |
| 203 | H | I | $OCF_3$ | H | I | 2 | |
| 204 | H | I | $OCF_3$ | Cl | Cl | 0 | |
| 205 | H | I | $OCF_3$ | Cl | Br | 0 | |
| 206 | H | I | $OCF_3$ | Cl | I | 0 | |
| 207 | H | I | $SCH_3$ | H | Cl | 0 | |
| 208 | H | I | $SCH_3$ | H | Br | 0 | |
| 209 | H | I | $SCH_3$ | H | I | 0 | |

Table 1 (continued)

| ($R^1 = CH_3$, $R^2 = H$) | | | | | | | |
|---|---|---|---|---|---|---|---|
| No. | $R^3$ | X | $Y^1$ | $Y^2$ | $Y^3$ | n | Melting point (°C) |
| 210 | H | I | $SCH_3$ | Cl | Cl | 0 | |
| 211 | H | I | $SCH_3$ | Cl | Br | 0 | |
| 212 | H | I | $SCH_3$ | Cl | I | 0 | |
| 213 | H | I | $SCHF_2$ | H | I | 0 | 76-79 |
| 214 | H | I | $SCHF_2$ | H | I | 1 | |
| 215 | H | I | $SCHF_2$ | H | I | 2 | |
| 216 | H | I | $N(CH_3)_2$ | H | I | 0 | 51-56 |
| 217 | H | I | $N(CH_3)_2$ | H | I | 1 | 73-77 |
| 218 | H | I | $N(CH_3)_2$ | H | I | 2 | |
| 219 | $CH_3$ | I | Cl | Cl | Cl | 0 | 159-161 |
| 220 | $CH_3$ | I | Cl | Cl | Cl | 1 | |
| 221 | $CH_3$ | I | Cl | Cl | Cl | 2 | |
| 222 | H | I | F | $CH_3$ | Cl | 0 | 100-102 |
| 223 | H | I | F | $CH_3$ | Cl | 1 | 198 |
| 224 | H | I | F | $CH_3$ | Cl | 2 | 228-231 |
| 225 | H | I | $CH_3$ | $CH_3$ | F | 0 | 190 |
| 226 | H | I | $CH_3$ | $CH_3$ | F | 1 | 97-99 |
| 227 | H | I | $CH_3$ | $CH_3$ | F | 2 | 209 |
| 228 | H | I | $OCH_3$ | Cl | Cl | 1 | 88 |
| 229 | H | I | $OCH_3$ | Cl | Cl | 2 | 83 |
| 230 | H | I | H | $CHF_2$ | Cl | 0 | 180 |
| 231 | H | I | H | $CHF_2$ | Cl | 1 | 163-166 |
| 232 | H | I | H | $CHF_2$ | Cl | 2 | 225 |
| 233 | H | I | I | H | F | 1 | 201 |
| 234 | H | I | I | H | F | 2 | 137 |
| 235 | H | I | $SCH_3$ | Cl | Cl | 0 | 146-147 |
| 236 | H | Cl | Cl | Cl | Cl | 0 | |
| 237 | H | I | Cl | Cl | Cl | 0 | |
| 238 | H | I | Cl | Cl | Cl | 1 | |
| 239 | H | I | Cl | Cl | Cl | 2 | |
| 240 | $CH_3$ | I | Cl | Cl | Cl | 0 | |
| 241 | $CH_3$ | I | Cl | Cl | Cl | 1 | |
| 242 | $CH_3$ | I | Cl | Cl | Cl | 2 | |
| 243 | H | I | H | Cl | F | 0 | |
| 244 | H | I | H | Cl | Cl | 0 | |
| 245 | H | I | H | Cl | Br | 0 | |
| 246 | H | I | H | Cl | I | 0 | |

Typical examples of the present invention are described below but they should not be construed as limiting the scope of the invention.

Example 1

Production of $N^2$-(1,1-dimethyl-2-methylthioethyl)-3-iodo-$N^1$-(4-iodo-2-methylphenyl)phthalamide (compound No. 30)

In 10 ml of acetonitrile was dissolved 1.5 g (4 mmol) of N-(1,1-dimethyl-2-methylthioethyl)-3-iodophthalisoimide, and 0.89 g (3.8 mmol) of 4-iodo-2-methylaniline was added to the solution. After 10 minutes, the crystals precipitated were collected by filtration to obtain 1.94 g of the desired compound.

Physical property: m.p. 155-157°C. Yield: 84%.

Example 2

Production of $N^2$-(1,1-dimethyl-2-methylsulfinylethyl)-3-iodo-$N^1$-(4-iodo-2-methylphenyl)phthalamide (compound No. 31)

In 10 ml of chloroform was dissolved 0.5 g (0.82 mmol) of $N^2$-(1,1-dimethyl-2-methylthioethyl)-3-iodo-$N^1$-(4-iodo-2-methylphenyl)phthalamide, after which the solution was cooled to 0°C and 0.19 g (1.1 mmol) of m-chloroperbenzoic acid was added to the solution. After stirring at room temperature for 1 hour, the reaction solution was poured into water and the desired compound was extracted with chloroform. The organic layer was washed with an aqueous sodium thiosulfate solution and 10% aqueous potassium carbonate solution, dried over anhydrous magnesium sulfate, and then distilled under reduced pressure to remove the solvent. The residue was washed with a small volume of ether to obtain 0.35 g of the desired compound.

Physical property: m.p. 99-102°C. Yield: 71%.

Example 3

Production of $N^2$-(1,1-dimethyl-2-methylsulfonylethyl)-3-iodo-$N^1$-(4-iodo-2-methylphenyl)phthalamide (compound No. 32)

In 10 ml of chloroform was dissolved 0.5 g (0.82 mmol) of $N^2$-(1,1-dimethyl-2-methylthioethyl)-3-iodo-$N^1$-(4-iodo-2-methylphenyl)phthalamide, and 0.47 g (2.72 mmol) of m-chloroperbenzoic acid was added to the solution. After stirring at room temperature for 3 hours, the reaction solution was poured into water and the desired compound was extracted with chloroform. The organic layer was washed with an aqueous sodium thiosulfate solution and 10% aqueous potassium carbonate solution, dried over anhydrous magnesium sulfate, and then distilled under reduced pressure to remove the solvent. The residue was washed with a small volume of ether to obtain 0.37 g of the desired compound.

Physical property: m.p. 116-118°C. Yield: 74%.

The agrohorticultural insecticide containing a phthalamide derivative represented by the formula (I) or salt thereof of the present invention as an active ingredient, are suitable for controlling various insect pests such as agrohorticultural insect pests, stored grain insect pests, sanitary insect pests, nematodes, etc., which are injurious to paddy rice, fruit trees, vegetables, other crops, flowers, ornamental plants, etc. They have a marked insecticidal effect, for example, on LEPIDOPTERA including summer fruit tortrix (Adoxophes orana fasciata), smaller tea tortrix (Adoxophyes sp.), Manchurian fruit moth (Grapholita inopinata), oriental fruit moth (Grapholita molesta), soybean pod border (Leguminovora glycinivorella), mulberry leafroller (Olethreutes mori), tea leafroller (Caloptilia thevivora), Caloptilia sp. (Caloptilia zachrysa), apple leafminer (Phyllonorycter ringoniella), pear barkminer (Spulerrina astaurota), common white (Piers rapae crucivora), tobacco budworm (Heliothis sp.), codling moth (Laspey resia pomonella), diamondback moth (Plutella xylostella), apple fruit moth (Argyresthia conjugella), peach fruit moth (Carposina niponensis), rice stem borer (Chilo suppressalis), rice leafroller (Cnaphalocrocis medinalis), tobacco moth (Ephestia elutella), mulberry pyralid (Glyphodes pyloalis), yellow rice borer (Scirpophaga incertulas), rice skipper (Parnara guttata), rice armyworm (Pseudaletia separata), pink borer (Sesamia inferens), common cutworm (Spodoptera litura), beet armyworm (Spodoptera exigua), etc.; HEMIPTERA including aster leafhopper (Macrosteles fascifrons), green rice leafhopper (Nephotettix cincticepts), brown rice planthopper (Nilaparvata lugens), whitebacked rice planthopper (Sogatella furcifera), citrus psylla (Diaphorina citri), grape whitefly (Aleurolibus taonabae), sweetpotato whitefly (Bemisia tabaci), greenhouse whitefly (Trialeurodes vaporariorum), turnup aphid (Lipaphis erysimi), green peach aphid (Myzus persicae), Indian wax scale (Ceroplastes ceriferus), cottony citrus scale (Pulvinaria aurantii), camphor scale (Pseudaonidia duplex), san Jose scale (Comstockaspis perniciosa), arrowhead scale (Unapsis yanonensis), etc.; TYLENCHIDA including soybean beetle (Anomala rufocuprea), Japanese beetle (Popillia japonica), tobacco beetle (Lasioderma serricorne), powderpost beetle (Lyctus brunneus), twenty-eight-spotted ladybird (Epilachna vigintiotopunctata), azuki bean weevil (Callosobruchus chinensis), vegetable weevil (Listroderes costirostris), maize weevil (Sitophilus zeamais), boll weevil (Anthonomus grandis grandis), rice water weevil (Lissorhoptrus oryzophilus), cucurbit leaf beetle (Aulacophora femoralis), rice leaf beetle (Oulema oryzae), striped flea beetle (Phyllotreta striolata), pine shoot beetle (Tomicus piniperda), Colorado potato beetle (Leptinotarsa decemlineata), Mexican bean beetle (Epilachna varivestis), corn rootworm (Diabrotica sp.), etc.; DIPTERA including (Dacus (Zeugodacus) cucurbitae), oriental fruit fly (Dacus(Bactrocera) dorsalis), rice leafminer (Agnomyza oryzae), onion maggot (Delia antiqua), seedcorn maggot (Delia platura), soybean pod gall midge (Asphondylia sp.), muscid fly (Musca domestica), house mosquito (Culex pipiens pipiens), etc.; and TYLENCHIDA including root-lesion nematode (Pratylenchus sp.), coffee root-lesion nematode (Pratylenchus coffeae), potato cyst nematode (Globodera rostochiensis), root-knot nematode (Meloidogyne sp.), citrus nematode (Tylenchulus semipenetrans), Aphelenchus sp. (Aphelenchus avenae), chrysanthemum foliar (Aphelenchoides ritzemabosi), etc.

The agrohorticultural insecticide, which contains as an active ingredient the phthalamide derivative of the general formula (I) or salt thereof of the present invention has a marked insecticidal effect on the above-exemplified insect pests injurious to paddy field crops, upland crops, fruit trees, vegetables, other crops, flowers and ornamental plants, and the like. Therefore, the desired effect of the agrohorticultural insecticide of the present invention can be obtained by applying the present agrohorticultural insecticide to the paddy field water, stalks and leaves of fruit trees, vegetables, other crops, flowers and ornamental plants, soil, etc., at a season at which the insect pests are expected to appear, before their appearance or at the time when their appearance is confirmed.

The agrohorticultural insecticide of the present invention is generally prepared into conveniently usable forms according to an ordinary manner for preparation of agrochemicals.

That is, the phthalamide derivative of the general formula (I) or a salt thereof and, optionally, an adjuvant are blended with a suitable inert carrier in a proper proportion and prepared into a suitable preparation form such as a suspension, emulsifiable concentrate, soluble concentrate, wettable powder, granules, dust, tablets, pack or the like through dissolution, dispersion, suspension, mixing, impregnation, adsorption or sticking.

The inert carrier usable in the present invention may be either solid or liquid. As a material usable as the solid carrier, there can be exemplified soybean flour, cereal flour, wood flour, bark flour, saw dust, powdered tobacco stalks, powdered walnut shells, bran, powdered cellulose, extraction residue of vegetables, powdered synthetic polymers or resins, clays (e.g. kaolin, bentonite, and acid clay), talcs (e.g. talc and pyrophyllite), silica powders or flakes (e.g. diatomaceous earth, silica sand, mica and white carbon [synthetic, high-dispersion silicic acid, also called finely divided hydrated silica or hydrated silicic acid, some of commercially available products contain calcium silicate as the major component]), activated carbon, powdered sulfur, pumice, calcined diatomaceous earth, ground brick, fly ash, sand, calcium carbonate, calcium phosphate and other inorganic or mineral powders, chemical fertilizers (e.g. ammonium sulfate, ammonium phosphate, ammonium nitrate, urea and ammonium chloride), and compost. These carriers may be used alone or as a mixture thereof.

A material usable as the liquid carrier is selected from materials that have solubility in themselves or which are without such solubility but are capable of dispersing an active ingredient with the aid of an adjuvant. The following are typical examples of the liquid carrier and can be used alone or as a mixture thereof: water, alcohols (e.g. methanol, ethanol, isopropanol, butanol and ethylene glycol), ketones (e.g. acetone, methyl ethyl ketone, methyl isobutyl ketone, diisobutyl ketone and cyclohexanone), ethers (e.g. ethyl ether, dioxane, Cellosolve, dipropyl ether and tetrahydrofuran), aliphatic hydrocarbon (e.g. kerosene and mineral oils), aromatic hydrocarbons (e.g. benzene, toluene, xylene, solvent naphtha and alkylnaphthalenes), halogenated hydrocarbons (e.g. dichloroethane, chloroform, carbon tetrachloride and chlorobenzene), esters (e.g. ethyl acetate, diisopropyl phthalate, dibutyl phthalate and dioctyl phthalate), amides (e.g. dimethylformamide, diethylformamide and dimethylacetamide), nitriles (e.g. acetonitrile), and dimethyl sulfoxide.

The following are typical examples of the adjuvant, which are used depending upon purposes and used alone or in combination is some cases, or need not be used at all.

To emulsify, disperse, dissolve and/or wet a compound as active ingredient, a surfactant is used. As the surfactant, there can be exemplified polyoxyethylene alkyl ethers, polyoxyethylene alkylaryl ethers, polyoxyethylene higher fatty acid esters, polyoxyethylene resonates, polyoxyethylene sorbitan monolaurate, polyoxyethylene sorbitan monooleate, alkylarylsulfonates, naphthalene sulfonic acid condensation products, ligninsulfonates and higher alcohol sulfate esters.

Further, to stabilize the dispersion of a compound as active ingredient, tackify it and/or bind it, the adjuvants exemplified below may also be used, namely, there may also be used adjuvants such as casein, gelatin, starch, methyl cellulose, carboxymethyl cellulose, gum arabic, poly(vinyl alcohol)s, turpentine, bran oil, bentonite and ligninsulfonates.

To improve the flowability of a solid product, the following adjuvants may also be used, namely, there may be used adjuvants such as waxes, stearates, alkyl phosphates, etc.

Adjuvants such as naphthalenesulfonic acid condensation products and polycondensates of phosphates may be used as a peptizer for dispersible products.

Adjuvants such as silicone oils may also be used as a defoaming agent.

Adjuvants such as 1,2-benzisothiazoline-3-one, 4-chloro-3,5-xylenol, butyl p-hydroxybenzoate may also be added as a preservative.

Further, if necessary, functional spreading agents, active enhancers such as metabolic decomposition inhibitor like piperonyl butoxide, anti-freezing agents such as propylene glycol, antioxidants such as BHT, ultraviolet absorbers, and the like may also be added.

The content of the compound as active ingredient may be varied as required, and the compound as active ingredient may be used in a proportion properly chosen in the range of 0.01 to 90 parts by weight per 100 parts of the agrohorticultural agent. For example, in dusts or granules, the suitable content of the compound as active ingredient is from 0.01 to 50 % by weight. In emulsifiable concentrates or flowable wettable powders, it is also from 0.01 to 50 % by weight.

The agrohorticultural insecticide of the present invention is used to control a variety of insect pests in the following manner: it is applied to a crop on which the insect pests are expected to appear, or a site where appearance or growth of the insect pests is undesirable, as it is or after being properly diluted with or suspended in water or the like, in an amount effective for control of the insect pests.

The applying dosage of the agrohorticultural insecticide of the present invention is varied depending upon various factors such as a purpose, insect pests to be controlled, a growth state of a plant, tendency of insect pests appearance, weather, environmental conditions, a preparation form, an application method, an application site and application time. It may be properly chosen in the range of 0.001 g to 10 kg, preferably 0.01 g to 1 kg, (in terms of the compound as active ingredient) per 10 ares depending upon purposes.

The agrohorticultural insecticide of the present invention may be used in admixture with other agrohorticultural insecticides, acaricides, nematocides, fungicides, biotic pesticides or the like in order to expand both spectrum of controllable insect pest species and the period of time when effective application are possible or to reduce the dosage. Furthermore, the agrohorticultural insecticide of the present invention may be used in admixture with herbicides, plant growth regulators, fertilizers or the like, depending upon application situations.

As the other agrohorticultural insecticides, acaricides and nematocides, which are used for the above purpose, there can be exemplified agrohorticultural insecticides, acaricides and nematocides, such as Ethion, Trichlorfon, Metamidophos, Acephate, Dichlorvos, Mevinphos, Monocrotophos, Malathion, Dimethoate, Formothion, Mecarbam, Vamidothion, Thiometon, Disulfoton, Oxydeprofos, Naled, Methylparathion, Fenitrothion, Cyanophos, Propaphos, Fenthion, Prothiofos, Profenofos, Isofenphos, Temephos, Phenthoate, Dimethylvinphos, Chlorfenvinphos, Tetrachlorvinphos, Phoxim, Isoxathion, Pyraclofos, Methidathion, Chlorpyrifos, Chlorpyrifos-methyl, Pyridaphenthion, Diazinon, Pirimiphosmethyl, Phosalone, Phosmet, Dioxabenzophos, Quinalphos, Terbuphos, Ethoprophos, Cadusafos, Mesulfenfos, DPS (NK-0795), Phosphocarb, Fenamiphos, Isoamidophos, Fosthiazate, Isazophos, Ethoprophos, Fenthion, Fostietane, Dichlofenthion, Thionazin, Sulprofos, Fensulfothion, Diamidafos, Pyrethrin, Allethrin, Prallethrin, Resmethrin, Permethrin, Tefluthrin, Bifenthrin, Fenpropathrin, Cypermethrin, α-Cypermethrin, Cyhalothrin, λ-Cyhalothrin, Deltamethrin, Acrinathrin, Fenvalerate, Esfenvalerate, Flucythrinate, Fluvalinate, Cycloprothrin, Ethofenprox, Halfenprox, Silafluofen, Fluvalinate, Methomyl, Oxamyl, Thiodicarb, Aldicarb, Alanycarb, Cartap, Metolcarb, Xylylcarb, Propoxur, Phenoxycarb, Fenobucarb, Ethiophencarb, Fenothiocarb, Bifenazate, BPMC, Carbaryl, Pirimicarb, Carbofuran, Carbosulfan, Furathiocarb, Benfuracarb, Aldoxycarb, Diafenthiuron, Diflubenzuron, Teflubenzuron, Hexaflumuron, Novaluron, Lufenuron, Flufenoxuron, Chlorfluazuron, Fenbutatin oxide, tricyclohexyltin hydroxide, sodium oleate, potassium oleate, Methoprene, Hydroprene, Binapacryl, Amitraz, Dicofol, Kersen, Chrorobenzilate, Bromopropylate, Tetradifon, Bensultap, Benzoximate, Tebufenozide, Methoxyfenozide, Chromafenozide, Propargite, Acequinosyl, Endosulfan, Diofenolan, Chlorfenapyl, Fenpyroximate, Tolfenpyrad, Fipronil, Tebufenpyrad, Triazamate, Etoxazole, Hexythiazox, nicotine sulfate, Nitenpyram, Acetamiprid, Thiacloprid, Imidacloprid, Thiamethoxam, Clothianidin, Nidinotefuran, Fluazinam, Pyriproxyfen, Hydramethylnon, Pyrimidifen, Pyridaben, Cyromazin, TPIC (tripropyl isocyanurate), Pymetrozin, Clofentezin, Buprofedin, Thiocyclam, Fenazaquin, Chinomethionate, Indoxacarb, Polynactin complexes, Milbemectin, Abamectin, Emamectin-benzoate, Spinosad, BT (Bacillus thuringiensis), Azadirachtin, Rotenone, hydroxypropyl starch, Levamisole hydrochloride, Metam-sodium, Morantel tartrate, Dazomet, Trichlamide, Pasteuria penetrans, Monacrosporium-phymatophagum, etc. As the agrohorticultural fungicides used for the same purpose as above, there can be exemplified agrohorticultural fungicides such as sulfur, lime sulfur, copper sulfate basic, Iprobenfos, Edifenfos, Tolclofos-methyl, Thiram, Polycarbamate, Zineb, Maneb, Mancozeb, Propineb, Thiophanate, Thiophanate methyl, Benomyl, Iminoctadin acetate, Iminocutadin albecylate, Mepronil, Flutolanil, Pencycuron, Furametpyl, Thifluzamide, Metalaxyl, Oxadixyl, Carpropamid, Dichlofluanid, Flusulfamide, Chlorothalonil, Kresoxim-methyl, Fenoxanil (NNF-9425), Himexazol, Etridiazol, Fluoroimide, Procymidone, Vinclozolin, Iprodione, Triadimefon, Triflumizole, Bitertanol, Ipconazole, Fluconazole, Propiconazole, Diphenoconazole, Myclobutanil, Tetraconazole, Hexaconazole, Tebuconazole, Imibenconazole, Prochloraz, Pefurazoate, Cyproconazole, Isoprothiolane, Fenarimol, Pyrimetanil, Mepanipyrim, Pyrifenox, Fluazinam, Triforine, Diclomezine, Azoxystrobin, Thiadiazin, Captan, Probenazole, Acibenzolar-S-methyl (CGA-245704), Fthalide, Tricyclazole, Pyroquilon, Chinomethionat, Oxolinic acid, Dithianon, Kasugamycin, Validamycin, Polyoxin, Blasticidin, Streptomycin, etc. Similarly, as the herbicides, there can be exemplified herbicides such as Glyphosate, Sulfosate, Glyfosinate, Bialaphos, Butamifos, Esprocarb, Prosulcarb, Benthiocarb, Pyributycarb, Asulam, Linulon, Dymron, Bensulfuron methyl, Cyclosulfamuron, Cinosulfuron, Pyrazosulfuron ethyl, Azimsulfuron, Imazosulfuron, Tenylchlor, Alachlor, Pretilachlor, Clomeprop, Etobenzanid, Mefenacet, Pendimethalin, Bifenox, Acifluorfen, Lactfen, Cyhalofop-butyl, Ioxynil, Bromobutide, Alloxydim, Setoxydim, Napropamide, Indanofan, Pyrazolate, Benzofenap, Pyraflufen-ethyl, Imazapyl, Sulfentrazone, Cafenstrole, Bentoxazon, Oxadiazon, Paraquat, Diquat, Pyriminobac, Simazine, Atrazine, Dimethametryn, Triazyflam, Benflesate, Flutiacet-methyl, Quizalofop-ethyl, Bentazon, calcium peroxide, etc.

As to the biotic pesticides, the same effect as above can be expected by using the agrohorticultural insecticide of the present invention in admixture with, for example, viral formulations obtained from nuclear polyhedrosis virus (NPV), granulosis virus (GV), cytoplasmic polyhedrosis virus (CPV), entomopox virus (EPV), etc.; microbial pesticides

utilized as insecticides or nematicides, such as Monacrosporium phymatophagum, Steinernema carpocapsae, Steinernema kushidai, Pasteuria penetrans, etc.; microbial pesticides utilized as fungicides, such as Trichoderma lignorum, Agrobacterium radiobactor, nonpathogenic Erwinia carotovora, Bacillus subtilis, etc.; and biotic pesticides utilized as herbicides, such as Xanthomonas campestris, etc.

In addition, the agrohorticultural insecticide of the present invention can be used in combination with biotic pesticides including natural enemies such as Parasitic wasp (Encarsia formosa), Parasitic wasp (Aphidius colemani), Gall-mildge (Aphidoletes aphidimyza), Parasitic wasp (Diglyphus isaea), Parasitic mite (Dacnusa sibirica), Predatory mite (Phytoseiulus persimilis), Predatory mite (Amblyseius cucumeris), Predatory bug (Orius sauteri), etc.; microbial pesticides such as Beauveria brongniartii, etc.; and pheromones such as (Z)-10-tetradecenyl=acetate, (E,Z)-4,10-tetradecadienyl= acetate, (Z)-8-dodecenyl=acetate, (Z)-11-tetradecenyl= acetate, (Z)-13-icosen-10-one, (Z)-8-dodecenyl= acetate, (Z)-11-tetradecenyl=acetate, (Z)-13-icosen-10-one, 14-methyl-1-octadecene, etc.

Typical examples of the present invention are described below but they should not be construed as limiting the scope of the invention.

As used in the examples, the terms "part" and "parts" are by weight.

| Formulation Example 1 | |
|---|---|
| Each compound listed in Table 1 or 2 | 10 parts |
| Xylene | 70 parts |
| N-methylprrolidone | 10 parts |
| Mixture of polyoxyethylene nonylphenyl ether and calcium alkylbenzenesulfonate | 10 parts |

An emulsifiable concentrate was prepared by mixing uniformly the above ingredients to effect dissolution.

| Formulation Example 2 | |
|---|---|
| Each compound listed in Table 1 or 2 | 3 parts |
| Clay powder | 82 parts |
| Diatomaceous earth powder | 15 parts |

A dust was prepared by mixing uniformly and grinding the above ingredients.

| Formulation Example 3 | |
|---|---|
| Each compound listed in Table 1 or 2 | 5 parts |
| Mixed powder of bentonite and clay | 90 parts |
| Calcium ligninsulfonate | 5 parts |

Granules were prepared by mixing the above ingredients uniformly, and kneading the resulting mixture together with a suitable amount of water, followed by granulation and drying.

| Formulation Example 4 | |
|---|---|
| Each compound listed in Table 1 or 2 | 20 parts |
| Mixture of kaolin and synthetic kaoline and high-dispersion silicic acid | 75 parts |
| Mixture of polyoxyethylene nonylphenyl ether and calcium alkylbenzenesulfonate | 5 parts |

A wettable powder was prepared by mixing uniformly and grinding the above ingredients.

Test Example 1: Insecticidal effect on diamond back moth (Plutella xylostella)

Adult diamond back moths were released and allowed to oviposit on a Chinese cabbage seedling. Two days after the release, the seedling having the eggs deposited thereon was immersed for about 30 seconds in a liquid chemical prepared by diluting a preparation containing each compound listed in Table 1 or 2 as an active ingredient

to adjust the prescribed concentration. After air-dryness, it was allowed to stand in a room thermostatted at 25°C. Six days after the immersion, the hatched insects were counted. The mortality was calculated according to the following equation and the insecticidal effect was judged according to the criterion shown below. The test was carried out with triplicate groups of 10 insects.

$$\text{Corrected mortality(\%)} = \frac{\text{Number of hatched insects in untreated group} - \text{Number of hatched insects in treated group}}{\text{Number of hatched insects in untreated group}} \times 100$$

Criterion:

A --- Mortality 100%
B --- Mortality 99-90%
C --- Mortality 89-80%
D --- Mortality 79-50%
E --- Mortality 49-0%

The compounds described in JP-A-2001-131141 were used as comparative compounds as follows:

A: $N^2$-(4-chloro-2-methylphenyl) -3-iodo-$N^1$-(1-methyl-2-methylthioethyl)phthalamide (compound No. 9)
B: $N^2$-(4-chloro-2-methylphenyl)-3-iodo-$N^1$-(2-ethylthio-1-methylethyl)phthalamide (compound No. 24)

The result is shown in Table 3 below.

Test Example 2: Insecticidal effect on Common cutworm (Spodoptera litura)

A piece of cabbage leaf (cultivar; Shikidori) was immersed for about 30 seconds in a liquid chemical prepared by diluting a preparation containing each compound listed in Table 1 or 2 as an active ingredient to adjust the prescribed concentration. After air-dryness, it was placed in a plastic Petri dish with a diameter of 9 cm and inoculated with second-instar larvae of common cutworm, after which the dish was closed and then allowed to stand in a room thermostatted at 25°C. Eight days after the inoculation, the dead and alive were counted. The mortality was calculated according to the following equation and the insecticidal effect was judged according to the criterion shown in Test Example 1. The test was carried out with triplicate groups of 10 insects.

$$\text{Corrected mortality(\%)} = \frac{\text{Number of alive larvae in untreated group} - \text{Number of alive larvae in treated group}}{\text{Number of alive larvae in untreated group}} \times 100$$

The result is shown in Table 3 below.

Table 3

| Test Compound No. | Concentration (ppm) | Test Example 1 | Test Example 2 |
|---|---|---|---|
| 2 | 50 | A | A |
| | 5 | A | E |
| 4 | 50 | A | A |
| | 5 | A | E |
| 5 | 50 | A | A |
| | 5 | A | A |
| 7 | 50 | A | A |
| | 5 | A | D |
| 19 | 50 | A | A |
| | 5 | A | E |
| 23 | 50 | A | E |
| | 5 | A | E |
| 24 | 50 | A | C |

Table 3 (continued)

| Test Compound No. | Concentration (ppm) | Test Example 1 | Test Example 2 |
|---|---|---|---|
| 25 | 5 | A | E |
|  | 50 | A | A |
| 26 | 5 | A | E |
|  | 50 | A | C |
| 27 | 5 | C | E |
|  | 50 | A | A |
| 28 | 5 | A | E |
|  | 50 | A | A |
| 29 | 5 | A | E |
|  | 50 | A | A |
| 30 | 5 | A | E |
|  | 50 | A | A |
| 31 | 5 | A | E |
|  | 50 | A | A |
| 32 | 5 | A | E |
|  | 50 | A | A |
| 42 | 5 | A | E |
|  | 50 | A | A |
| 43 | 5 | A | A |
|  | 50 | A | A |
| 44 | 5 | A | E |
|  | 50 | A | A |
| 56 | 5 | A | C |
|  | 50 | A | A |
| 57 | 5 | A | E |
|  | 50 | A | E |
| 58 | 5 | A | E |
|  | 50 | A | A |
| 84 | 5 | A | E |
|  | 50 | A | A |
| 99 | 5 | A | E |
|  | 50 | E | A |
| 100 | 5 | A | E |
|  | 50 | A | A |
| 101 | 5 | A | E |
|  | 50 | A | - |
| 102 | 5 | A | - |
|  | 50 | A | A |
| 103 | 5 | A | A |
|  | 50 | A | A |
| 104 | 5 | A | E |
|  | 50 | A | A |
| 105 | 5 | A | E |
|  | 50 | A | A |
| 106 | 5 | A | E |
|  | 50 | A | A |
| 107 | 5 | A | E |
|  | 50 | A | A |
| 117 | 5 | A | E |
|  | 50 | A | A |

Table 3 (continued)

| Test Compound No. | Concentration (ppm) | Test Example 1 | Test Example 2 |
|---|---|---|---|
| | 5 | A | A |
| 118 | 50 | A | A |
| | 5 | A | A |
| 119 | 50 | A | A |
| | 5 | A | A |
| 123 | 50 | A | A |
| | 5 | A | A |
| 124 | 50 | A | A |
| | 5 | A | E |
| 125 | 50 | A | A |
| | 5 | A | E |
| 142 | 50 | A | A |
| 143 | 5 | A | A |
| | 50 | A | A |
| 144 | 5 | A | E |
| | 50 | A | D |
| 147 | 5 | A | D |
| | 50 | A | C |
| 148 | 5 | A | E |
| | 50 | A | A |
| 149 | 5 | A | E |
| | 50 | A | A |
| 153 | 5 | A | E |
| | 50 | A | A |
| 201 | 5 | A | E |
| | 50 | A | A |
| 202 | 5 | A | E |
| | 50 | A | E |
| | 5 | A | E |
| A | 50 | A | E |
| | 5 | E | E |
| B | 50 | A | E |
| | 5 | E | E |

Test Example 3

Controlling effect on diamondback moth (Plutella xylostella) by soil treatment for cabbage

At the time of trans-planting cabbage plants (cultivar: YR Seitoku), planting holes were treated with granules prepared from the compound listed in Table 1 or 2 according to Formulation Example. Diamondback moths parasitic on the cabbage plants were counted on predetermined days after the trans-planting. The results are shown in Table 4. In Table 4, mgAI/plant denotes the dosage (mg) of the active ingredient per plant.

Table 4

| Test Agent (Test Compound No.) | Dosage (mgAI/plant) | Number of parasites/3 plants | | | |
|---|---|---|---|---|---|
| | | After 18 days | After 25 days | After 32 days | After 42 days |
| 25 | 20 | 0 | 0 | 6 | 22 |
| 28 | 20 | 0 | 7 | 23 | 59 |

Table 4 (continued)

| Test Agent (Test Compound No.) | Dosage (mgAI/plant) | Number of parasites/3 plants | | | |
|---|---|---|---|---|---|
| | | After 18days | After 25days | After 32days | After 42days |
| 30 | 20 | 0 | 9 | 19 | 37 |
| A (Compound No.9 of JP-A-2001-131141) | 20 | 8 | 75 | 143 | 146 |
| Untreated | - | 125 | 182 | 204 | 235 |

## Claims

**1.** A phthalamide derivative represented by general formula (I):

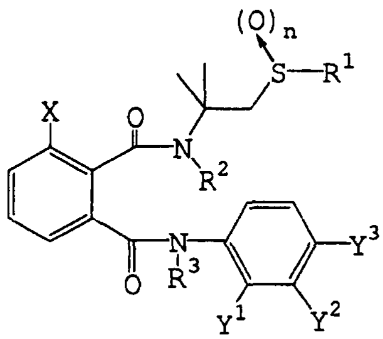

(I)

wherein each of $R^1$, $R^2$ and $R^3$, which may be the same or different, is a hydrogen atom, a $(C_1-C_6)$alkyl group, a $(C_3-C_6)$alkenyl group or a $(C_3-C_6)$alkynyl group; each of X and $Y^3$, which may be the same or different, is a halogen atom; each of $Y^1$ and $Y^2$, which may be the same or different, is a hydrogen atom, a halogen atom, a nitro group, a cyano group, a $(C_1-C_6)$alkyl group, halo$(C_1-C_6)$alkyl group, a $(C_2-C_6)$alkenyl group, a halo$(C_2-C_6)$alkenyl group, a $(C_2-C_6)$alkynyl group, a halo$(C_2-C_6)$alkynyl group, a $(C_3-C_6)$cycloalkyl group, a halo$(C_3-C_6)$cycloalkyl group, a $(C_1-C_6)$alkoxy group, halo$(C_1-C_6)$alkoxy group, a $(C_1-C_6)$alkylthio group, halo$(C_1-C_6)$alkylthio group, a $(C_1-C_6)$alkylsulfinyl group, halo $(C_1-C_6)$alkylsulfinyl group, a $(C_1-C_6)$alkylsulfonyl group, halo$(C_1-C_6)$alkylsulfonyl group, a mono$(C_1-C_6)$alkylamino group or a di$(C_1-C_6)$alkylamino group whose $(C_1-C_6)$alkyl groups may be the same or different; and n is an integer of 0 to 2.

**2.** A phthalamide derivative according to claim 1, wherein $R^1$ is a $(C_1-C_6)$alkyl group; each of $R^2$ and $R^3$ is a hydrogen atom; each of X and $Y^3$, which may be the same or different, is a halogen atom; $Y^1$ is a halogen atom or a $(C_1-C_6)$ alkyl group; $Y^2$ is a halogen atom; and n is an integer of 0 to 2.

**3.** An agricultural and horticultural insecticide **characterized by** containing a phthalamide derivative according to claim 1 or 2 as an active ingredient.

**4.** A method for applying an agricultural and horticultural insecticide, **characterized by** treating a plant to be protected or soil with an effective amount of an agricultural and horticultural insecticide according to claim 3 in order to protect useful plants against insect pests.

## INTERNATIONAL SEARCH REPORT

International application No.
PCT/JP02/04172

**A. CLASSIFICATION OF SUBJECT MATTER**

Int.Cl$^7$ C07C323/42, 317/28, 317/40, C07D213/64, A01N37/22, 41/10, 43/40

According to International Patent Classification (IPC) or to both national classification and IPC

**B. FIELDS SEARCHED**

Minimum documentation searched (classification system followed by classification symbols)

Int.Cl$^7$ C07C323/00, 317/00, C07D213/00, A01N37/00, 41/00, 43/00

Documentation searched other than minimum documentation to the extent that such documents are included in the fields searched

Electronic data base consulted during the international search (name of data base and, where practicable, search terms used)

REGISTRY(STN), CA(STN)

**C. DOCUMENTS CONSIDERED TO BE RELEVANT**

| Category* | Citation of document, with indication, where appropriate, of the relevant passages | Relevant to claim No. |
|---|---|---|
| X | EP 1006107 A2 (Nihon Nohyaku Co.), 07 June, 2000 (07.06.00), & JP 2001-131141 A | 1-4 |
| A | EP 919542 A2 (Nihon Nohyaku Co.), 02 June, 1999 (02.06.99), & JP 11-240857 A | 1-4 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 July, 2002 (30.07.02) | 13 August, 2002 (13.08.02) |

| Name and mailing address of the ISA/ | Authorized officer |
|---|---|
| Japanese Patent Office | |
| Facsimile No. | Telephone No. |

Form PCT/ISA/210 (second sheet) (July 1998)